# EUROPÄISCHE PATENTANMELDUNG

(11) **EP 0 857 974 A1**
(43) Veröffentlichungstag der Anmeldung: **12.08.1998**
(21) Anmeldenummer: 98102387.2
(22) Anmeldetag: 11.02.1998
(51) Int. Cl.: G01N 33/74, G01N 33/53

(54) **Schnelltest zur Progesteronbestimmung in Milch**

(30) Priorität: 11.02.1997 DE 19705163
(71) Anmelder: BIOLAB GMBH, D-80995 München (DE)
(72) Erfinder: Arnstadt, Ingo, Dr., 80995 München (DE)
(74) Vertreter: Fürniss, Peter, Dr.

(57) **Zusammenfassung**

Das bekannte Verfahren zur qualitativen/semiquantitativen Bestimmung von Progesteron in einer biologischen Flüssigkeit nach einem ELISA -Prinzip enthält folgende Schritte:

Man entnimmt einige Tropfen der zu untersuchenden Flüssigkeit, verdünnt sie mit einer wäßrigen Probenverdünnerlösung, bringt diese Mischung mit einer mit spezifischem Antikörper beschichteten Oberfläche in Berührung und gibt eine Progesteron-Enzymkonjugat-Lösung hinzu, die vorzugsweise mit noch freien Bindungsstellen der Antikörper reagiert, die an einer Oberfläche fixiert sind. Inkubationsmischung und ungebundene Reaktionspartner werden durch das Auswaschen von der Oberfläche entfernt und das gebundene Enzym mittels Farbreaktion nachgewiesen. Die entwickelte Farbe ist ein Maß für den Progesterongehalt der zu untersuchenden Flüssigkeit.

Das bekannte Verfahren eignet sich insbesondere zu einem Schnelltest für die einfache und zuverlässige Ermittlung von Besamungs- bzw. Befruchtungszeitpunkt und Kontrolle der Trächtigkeit bei Haustieren. Damit werden erstmals Östrus- und Trächtigkeitsbereich mit hoher Zuverlässigkeit vom Zwischenbereich niedriger Progesteronkonzentrationen abgegrenzt und zahlreiche, mit bisherigen Verfahren üblicherweise erstellte Fehldiagnosen und Fehlinterpretationen in hohem Maße vermieden. Dieses bekannte Verfahren kann bei gleicher Genauigkeit und Zuverlässigkeit wesentlich schneller durchgeführt werden, wenn man die reagierenden Flüssigkeiten der Schritte iii und vi vor ihrer Vereinigung so erwärmt, daß das genannte Reaktionsgemisch am Beginn der betreffenden Inkubationsschritte eine Temperatur von 33 ± 5 °C besitzt.

## Beschreibung

Die Erfindung betrifft einen Schnelltest zur qualitativen/semiquantitativen Bestimmung von Progesteron in einer Flüssigkeit nach einem ELISA-Prinzip gemäß Patentanspruch 1. Die Erfindung betrifft weiterhin eine Testpackung zur Durchführung des genannten Schnelltests nach Patentanspruch 4. Die vorliegende Erfindung stellt eine Weiterentwicklung des in dem deutschen Gebrauchsmuster G 92 16 110.3 und dem europäischen Patent EP 0 671 006 der Anmelderin beschriebenen immunologischen Schnelltests zur optischvisuellen Bestimmung des Hormons Progesteron in Milch und Blut dar. Der Inhalt der genannten Druckschriften wird daher auch zum Inhalt der vorliegenden Patentanmeldung gemacht.

Der bekannte Schnelltest bietet als erster seiner Art eine ausreichende Genauigkeit der Testaussage. Zur Erreichung dieses Ziels war die Einführung eines Emulsions-/Verdünnungsschrittes mit einer gewissen Reaktionszeit von einigen Minuten erforderlich, wodurch der Test naturgemäß verlängert wird.

Die Gesamtinkubationszeiten jenes bekannten Tests betragen beispielsweise 9-13 Minuten. Die Inkubationszeiten bekannter Schnelltests, sind in Tabelle 1 zusammengestellt. Sie enthält den Schnelltest nach EP 0 671 006 unter der Bezeichnung Hormonost® Schnelltest (alt). Aus Tab. 1 geht hervor, daß sich dessen Zeitbedarf im Mittelfeld von insgesamt acht Schnelltests bewegt.

Auch wenn die Richtigkeit der Testaussage prinzipiell höher als die Schnelligkeit eines Tests zu werten ist, ist der aufzuwendende Zeitbedarf für seine praktische Anwendung, z.B. durch den Tierhalter auf dem Bauernhof ("On-the-farm"-Test), von größter Wichtigkeit. Hierdurch werden letztendlich dessen Akzeptanz und Anwendungshäufigkeit bestimmt.

Der Erfindung liegt daher die Aufgabe zugrunde, den Zeitbedarf für den bekannten Test Hormonost® Schnelltest unter einfachen Praxisbedingungen, wie bei der Durchführung auf einem Bauernhof, zu reduzieren ohne daß die relativ große Zuverlässigkeit der Testaussage verloren geht, die in der unten stehenden Tabelle 1, Spalte "Trefferquote" vergleichend angegeben ist. Diese Aufgabe wird mit dem Schnelltest gemäß Anspruch 1 bzw. mit der Testpackung für den genannten Schnelltest gemäß Anspruch 4 gelöst. Die Unteransprüche nennen bevorzugte Ausführungsformen der vorliegenden Erfindung.

Als Kleinthermostat a) kann gemäß einer bevorzugten Ausführungsform ein handelsüblicher Haushaltsthermostat eingesetzt werden, wie er beispielsweise in Form eines Babyflaschenwärmers in großer Stückzahl zu vergleichsweise geringen Kosten hergestellt und vertrieben wird. Als Kurzzeitwecker b) kann vorzugsweise ein normalerweise im Haushalt verwendeter preiswerter Kurzzeitwecker verwendet werden.

Der Thermostat a) dient zum Aufwärmen der Reagenzienlösung. Da beim Progesteron-Enzym-Konjugat (e) und beim Chromogen (g) in der Wärme die Stabilität leiden kann, können von der Reagenzienlösung nur die Probenverdünner-Lösung (d) und die Substratpuffer-Lösung (f) aufgewärmt werden. Diese stellen einerseits den größeren Anteil des Röhrchenvolumens im Test dar und sind andererseits stabil genug gegenüber mehrfachem Aufwärmen eines Testkit-Inhalts zur jeweiligen Testdurchführung.

Es wird also nicht wie allgemein üblich die Inkubationsmischung im Reaktionsgefäß, was in diesem speziellen Fall das mit Progesteron-Antikörpern beschichtete Teströhrchen ist, direkt einer höheren Temperatur zur Reaktionsbeschleunigung ausgesetzt. Denn hiermit ist die potentielle Gefahr einer partiellen Beeinträchtigung der adsorbierten Antikörper verbunden, was die Reproduzierbarkeit der Testergebnisse verringern würde. Die Reaktionstemperatur wird nur indirekt durch Vorwärmen eines Teils der Reagenzlösungen (hier: zweier Flaschen auf 55 ± 10 °C) erhöht. Der Test läuft anschließend im Teströhrchen bei 33 ± 5 °C anstatt bisher bei Raumtemperatur, (meist 20 ± 2 °C) und daher entsprechend schneller ab. Infolge des Temperaturgradienten zwischen Röhrchenfüllung und äußerer Raumtemperatur sinkt natürlich die Reaktionstemperatur im Laufe der Inkubation, aber die anfänglich erhöhte Starttemperatur bewirkt bereits, daß der Zeitbedarf für sämtliche Reaktionsschritte auf mehr als die Hälfte verkürzt wird.

Der Kurzzeitwecker b) mit Sekundenanzeige erleichert die exakte Einhaltung der Inkubationszeiten, was wegen des schnellen Reaktionsablaufs erforderlich ist.

Der Vorteil dieses Tests liegt einmal in der offensichtlichen Zeitersparnis gegenüber der bisherigen Testausführung von Hormonost®. Nach den untenstehend aufgeführten Beispielen können die Gesamtinkubationszeiten von bisher 9 - 13 min. (bevorzugte Testausführung 13 min.) auf 2,5 - 5 min. (bevorzugte Ausführungsform 4 min.) reduziert werden.

**Tabelle 1**

| Testkit (mit Literaturangab) | Hersteller | Vertrieb für Deutschland | Trefferquote (%) | Inkubationszeit in Minuten |
|---|---|---|---|---|
| Ovucheck® Praxistest (1) Rapid WELL Kit | Cambridge Vet.Sci., UK | Smith Kline GmbH | 70,5 | 8-10 |
| Progestassay® Milchprogesterontest (1) | Pitman-Moore, USA | Janssen GmbH | 72,5 | 15-20 |
| Reprostrip-Progesteron-Schnelltest (1) | Nochtech, Irland | A. Albrecht GmbH & Co. KG | 70,9 | 10 |
| Enzygnost® Milchprogesteron (1) | Hoechst IQ (Bio) UK | Hoechst Veterinär GmbH | 73,9 | 27 |
| Bovitest® (2) | Santel, Frankreich | Dr. Berger GmbH & Co.KG | 80 und 39,2 (!) | 10 |
| Hygia® RPT (3) | ImmuCell USA | Hartmann AG | 73,5 | 4 |
| Target® | BioMetallios, USA | A.Albrecht GmbH & Co. KG | 73,5 | 8 (alt) 12 (neu) |
| Hormonost® (3) Schnelltest | Biolab GmbH München | Biolab GmbH München | 91,0 | 9-13 (alt) 4 (neu) |

| | | | | |
|---|---|---|---|---|
| Literatur 1) Sobiraj, A. et al., Tierärztl. Praxis 17 (1989) 21-25 | | | | |
| 2) Saner, R., Zeitschrift Zuchthygiene 23 (1988) 113 | | | | |
| 3) Sobiraj, A. et al., Tierärztl. Praxis 23 (1995), 32-36 | | | | |

Nach Tab. 1 würde der erfindungsgemäße Schnelltest (Hormonost® Schnelltest (neu)) somit die Testdauer des bisher schnellsten Progesterontests Hygia® von ImmuCell® erreichen. Dieser hat jedoch den offensichtlichen Nachteil relativ großer Unzuverlässigkeit von durchschnittlich mehr als 25 % Fehlresulaten. Ferner wurde zu diesem Test die Benutzung eines mitgelieferten Taschenphotometers empfohlen, was die Definition "visueller Schnelltest" einerseits fragwürdig macht, aber andererseits die effektive Testzeit verlängert. Dagegen sind beim erfindungsgemäßen Schnelltest die Farbunterschiede auch für das unbewaffnete menschliche Auge gut und mit zuverlässig sicherer Testaussage zu erkennen.

Mit der beschriebenen Testanordnung wird der Nachteil einer längeren Testdauer infolge des einleitend erwähnten Emulsions-/Verdünnungsschrittes nicht nur kompensiert, sondern der Zeiteinsparungseffekt geht darüber hinaus, so daß der Schnelltest nunmehr in weniger als der Hälfe des bisherigen Zeitbedarfs ausgeführt werden kann.

Ein weiterer und wesentlicher Vorteil der neuen Testanordnung besteht darin, daß die Vorbereitungs- bzw. Anwärmzeit der Testreagenzien vor allem zeitsparend verkürzt wird. Bisherige Vefahrensweisen von Progesteronschnelltests ("on-the-farm-tests") haben einen gewichtigen Nachteil, der die Vorbereitung der Testreagenzien im sog. Testkit mit sich bringt. Diese werden üblicherweise im Kühlschrank aufbewahrt und sollen jeweils vor Gebrauch auf die zur Testausführung notwendigen Raumtemperatur gebracht werden. Hierfür werden Mindest-Anwärmzeiten von beispielsweise 1-24 Stunden von den Herstellern vorgegeben. Entsprechend unserer Testanordnung kann die eigentliche Testdurchführung statt nach mindestens 60 min. schon nach 5 min. begonnen werden. Das entspricht der Zeit, die man ohnehin für die Vorbereitung der Teströhrchen und das Einpipettieren der zu untersuchenden Milch oder des Serums benötigt.

Ein zusätzlicher Vorteil der erfindungsgemäßen Testanordnung besteht darin, daß die Vorbereitungs- und Anwärmzeit der Testreagenzien exakt definiert wird und der Test selber dadurch reproduzierbarer und zuverlässiger ausgeführt werden kann, auch unter schwierigen Bedingungen. Eine von den erhofften Anwendungen eines Progesteronschnelltests für Milchproben her stammende Bezeichnung lautet "Stallgassentest" (Möller, R. u. W. Holtz, "Der Tierzüchter", 41 (1989) 156-157; Sobiraj et al. "top agrar" 6/1993, R12-R14). Vom praktischen Arbeitsablauf einer der häufigsten Anwendungsformen dieses Testprinzips wäre die Durchführung während der Tätigkeit des Tierhalters im Milchviehstall wünschenswert. Tatsächlich ist dies aber mit Fehlerrisiken verbunden, weil die Temperatur mitteleuropäischer Ställe inklusive der anschließenden Arbeitsräumen (Melkkammer) gerade in der kühlen Jahreszeit mit ca. 9 ± 3 °C weit unterhalb der zur fehlerfreien Testdurchführung notwendigen Temperatur von mindestens 20 ± 2 °C liegt. Dies ist umso gravierender als in diese Jahreszeit der größte Anteil an Fruchtbarkeitsmaßnahmen inkl. Besamungen fällt und somit auch der Bedarf an einem solchen Progesteronschnelltest. Mit der beschriebenen Testanordnung kann diese wichtige Anwendungsmöglichkeit ebenfalls fehlerfrei erschlossen werden.

Eine derartige einfache Lösung der Aufgabe, besonders für die Anwendung in der Landwirtschaft oder einfachen Tierarztpraxis, also im Nichtlaborbereich, war bisher völlig unbekannt, obwohl Progesteron-Schnelltests seit mindestens 12 Jahren auf dem Markt sind und zahlreiche Weiterentwicklungen erfahren haben. Jedoch wurden bei den modifizierten bekannten Tests die Inkubationszeiten verlängert (z.B. von 8 auf 12 min. beim Test Target® in der neusten Version), was zweifellos der Aufgabe der vorliegenden Erfindung widerspricht.

### Praktische Durchführung der visuellen Progesteronbestimmung:

**1.** Vorbereitung
   **1.0.** Heißes Leitungswasser (ca. 40-60°C) bis fast zum Rand des Kleinthermostat einfüllen. Vorher geeichte Heizstufe einschalten, die bei ca. 55°C die Heizung unterbricht.
   **1.1.** Testkit aus dem Kühlschrank nehmen. Fläschen Nr. 1 (Probenverdünner) und Nr. 3 (Substrat) 5 Minuten in das Wasserbad des Thermostaten (oder einer anderweitig auf 55 ± 10 °C gebrachten Heizflüssigkeit) bringen. Kurzwecker auf 5 min. einschalten.
   **1.2.** Gestell mit Röhrchen vorbereiten. Es werden eine oder mehrere Milchproben genommen. Wegen der erforderlichen Referenzfärbung wird bei jedem Testlauf ein Standard und bei Bedarf zusätzlich eine Kontrolle mitgemessen. Diese werden den Flaschen "Standardlösung Brunst" und einer Kontrollmilch "Trächtig" entnommen.
**2**). Testausführung (Beispiele 1 bis 3 für Milchproben)
   **2.0.** Drei Tropfen (= 120 ± 40 µl) Standard- oder Probenmilch (Beispiel 2: 5 Tropfen, Beispiel 3: 2 Tropfen) werden in den unteren Teil des jeweiligen Röhrchens gegeben. Protokollnotizen eintragen.
   **2.1.** Nach Ablauf von 5 min. Anwärmezeit, die auf 10-15 min. verlängert werden kann, Fläschen Nr. 1 (Probenverdünner, ca. 55 °C) dem Wasserbad entnehmen, auf Papiertuch vortropfen. 5 Tropfen (= 370 ± 40 µl) Probenverdünner (Beispiel 2: 3 Tropfen (210 ± 30 µl), Beispiel 3: 5 Tropfen) werden in jedes Röhrchen zugetropft. Gegebenenfalls auf gleichen Flüssigkeitsstand nachtüpfeln. 10 sec. seitlich schütteln und insgesamt 0,5 min. (Beispiel 2:1 min, Beispiel 3: 2 min.) reagieren lassen. Die Temperatur der Inkubationsmischung wird durch die Zugabe des Probenverdünners bestimmt. Bei Start dieses Verdünnungsschrittes beträgt sie ca. 35 ± 3 °C (Beispiel 2: ca. 28 ± 2°C, Beispiel 3: ca. 30 ± 2 °C).
   **2.2.** Nach Ablauf der mittels Kurzzeitwecker eingestellten Zeit wird 1 Tropfen (65 ± 20 µl) von Fläschchen Nr. 2 (Enzym = Progesteron-Enzym-Konjugat) in jedes Röhrchen gegeben (Beispiel 2 und 3: 2 Tropfen, 115 ± 30 µl) kurz aufschütteln und insgesamt 1 min. reagieren lassen (Beispiel 2: 1 min, Beispiel 3: 2 min); Starttemperaturen: Beispiel 1 ca. 26 + 1 °C, Beispiel 2 und 3: ca. 25 ± 2 °C)
   **2.3.** Nach Ablauf der eingestellten Zeit von 1 bzw. 2 min. den Röhrcheninhalt ausleeren, rasch 4 x mit kaltem Leitungswasser nachwaschen. Zuletzt das Gestell mit den Röhrchen per Hand ausschleudern.
   **2.4.** Fläschen Nr. 3 (Substrat, ca. 60 °C) dem Wasserbad entnehmen und 6-10 Tropfen (500 ± 150 µl) Substrat (= Substratpufferlösung) pro Röhrchen zugeben, bis bei allen Röhrchen jeweils der gleiche Flüssigkeitsstand erreicht ist.
   **2.5.** 2 Tropfen (80 ± 20 µl) von Fläschen Nr. 4 (Anfärber = Chromogen) rasch hintereinander zugeben (Beispiel 2 und 3: 1 Tropfen (55 ± 15 µl)). Kurz durchmischen. Die Starttemperatur dieser Farbreaktion beträgt hier 30 ± 2 °C.

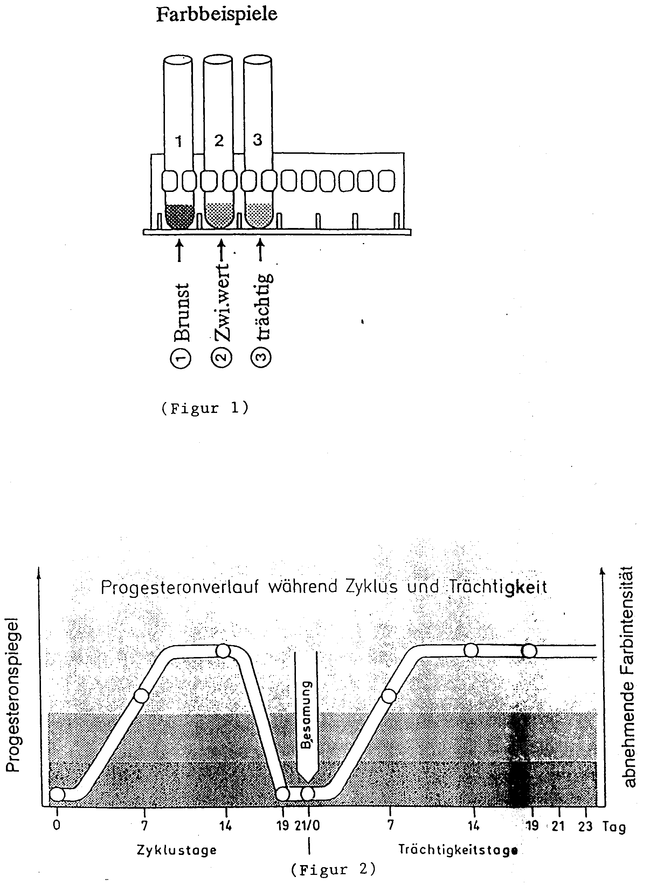

Nach 1 min. (Beispiel 2 und 3: 2 min.) - sobald genügend Farbe entwickelt ist - die Färbung der Proben im Vergleich zum Stardard bzw. Kontrollröhrchen ablesen und beurteilen. Die Farbbeurteilung eventuell 2-3 min. später nachkontrollieren. Es werden zur Auswertung unterschieden (vgl. Fig. 1 und 2):

### Auswertung und Testbeurteilung:

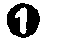 Kräftige Farbe - Progesterongehalt niedrig
   - ○: Färbung leicht oder dunkler als Standard: "Brunst" Kein Progesteron bzw. kein aktiver Gelbkörper vorhanden.
   - ○: Kann Brunst oder Brunstnähe bedeuten, wenn zusätzliche äußere Brunstsymptome festzustellen sind.
   - ○: Eindeutige Aussage: Die Kuh ist nicht trächtig.
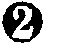 **Mittlere Färbung Progesteronwert im Zwischenbereich**
   - ○: Färbung heller als Standardröhrchen "Brunst" aber dunkler als Kontrollröhrchen "Trächtig": Gelbkörperanbildung oder -rückbildung. Progesteron im Zwischenbereich.
   - ○: Kann Brunstnähe bedeuten. In diesem Fall befindet sich die Kuh 2-3 Tage vor oder 3-4 Tage nach der Brunst. Eine Probenmessung 1-2 Tage später zeigt, ob der Zyklus in Richtung Brunst oder Zyklusmitte weiter fortgeschritten ist. Im ersten Fall steht eine Besamung an oder unmittelbar bevor. Im zweiten Fall Datum für gezielte Brunstbeobachtung beginnend in 16 Tagen notieren.
   - ○: Eindeutige Aussage: Die Kuh ist weder in Brunst, noch ist sie trächtig.
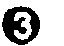 **Schwache Färbung Progesterongehalt hoch**
   - ○: Färbung gleich oder heller als Kontrollmilch "Trächtig": Aktiver Gelbkörper vorhanden. (In Ausnahmefällen kann die Färbung bei trächtigen Tieren am Tag 21 auch leicht dunkler als Kontrollmilch "Trächtig" ausfallen. Bei Unsicherheit sollte der Test nach 2 Tagen wiederholt werden).
   - ○: Kann Frühträchtigkeit z.B., 19-23 Tage nach Besamung bedeuten. Bevorzugte Tage für diese Untersuchung mittels Progesterontest sind Tage 20-21. Der Hinweis auf Trächtigkeit ist stets ab 6.- 8. Woche nach Besamung durch eine tierärztliche Untersuchung zu bestätigen, um einen eventuell inzwischen erfolgten Embryonentod auszuschließen (ca. 5-10% der Frühträchtigkeiten).
   - ○: Eindeutige Aussage: Die Kuh ist nicht in Brunst.

## Patentansprüche

1. Schnelltest zur qualitativen/semiquantitativen Bestimmung von Progesteron in Milch nach einem ELISA-Prinzip, wobei man
i) einige Tropfen der zu untersuchenden Flüssigkeit nimmt,
ii) diese mit einer mit einem spezifischen Antikörper beschichteten Oberfläche in Berührung bringt,
iii) die Flüssigkeit mit einer wäßrigen Probenverdünnerlösung versetzt,
iv) eine Progesteron-Enzymkonjugat-Lösung hinzugibt, die vorzugsweise mit den noch freien Bindungsstellen der fixierten Antikörper reagiert,
v) die Mischung der Lösungen, d. h. die nicht vom Antikörper gebundenen Substanzen, entfernt,
vi) das gebundene Enzym durch eine Farbreaktion nachweist und anhand der visuell beurteilten Intensität der entstehenden Farbe den Progesterongehalt der zu untersuchenden Flüssigkeit bestimmt,
wobei man die Schritte ii) und iii) auch in umgekehrter Reihenfolge oder gleichzeitig durchführen kann.
**dadurch gekennzeichnet,**
daß man die reagierenden Flüssigkeiten der Schritte iii) und vi) vor ihrer Vereinigung so erwärmt, daß das jeweilige Reaktionsgemisch anfänglich eine Temperatur von 33 ± 5 °C besitzt.

2. Schnelltest nach Anspruch 1,
**dadurch gekennzeichnet,**
daß man die Probenverdünnerlösung für den Schritt iii) und die Substratpufferlösung für den Schritt vi) vor der jeweiligen Zugabe auf 55 ± 10 °C erwärmt.

3. Schnelltest nach Anspruch 1,
**dadurch gekennzeichnet,**
daß man zum optischen Nachweis des Enzyms eine auf 55 ± 10°C erwärmte Substratpufferlösung einsetzt, die bereits ein Substrat enthält und der als zweites Substrat eine Chromogenlösung hinzugefügt wird.

4. Testpackung für einen qualitativen/semiquantitativen Schnelltest auf Progesteron in einer Flüssigkeit nach dem ELISA-Prinzip, enthaltend
a) einen Kleinthermostat
b) einen Kurzzeitwecker mit Sekundenanzeige
c) Gefäße mit einer mit anti-Progesteron-Antikörpern bzw. anti-Progesteron-IgG beschichteten Oberfläche,
und mindestens je einen Behälter mit
d) Probenverdünnerlösung und
e) Progesteron-Enzymkonjugat-Lösung, sowie
f) mindestens ein Substrat oder eine Substratmischung zum optischen Nachweis des Enzyms durch die Farbreaktion.

5. Testpackung nach Anspruch 4,
**dadurch gekennzeichnet,**
daß die Substratmischung eine Substratpuffer-Lösung f) und eine Chromogenlösung g) enthält

6. Testpackung nach Anspruch 4 und/oder 5,
**dadurch gekennzeichnet daß**
sie zusätzlich je einen Behälter mit
h) Standardprobe bzw. Standardlösung "Brunst" ("Östrus") und
i) Kontrollprobe "Trächtig" (Corpus Luteum aktiv, "Ovulation") enthält.

7. Testpackung nach einem der Ansprüche 4 bis 6,
**dadurch gekennzeichnet daß**
sie zusätzlich
k) Einmal-Pipetten und
l) ein Röhrchengestell enthält.

8. Testpackung nach einem der Ansprüche 4 bis 7
**gekennzeichnet durch**
m) einen Behälter mit einer Stop-Lösung zur Beendigung der Farbreaktion und Fixierung der Farbintensität für deren nachfolgende photometrische Messung und somit Dokumentation der Ergebnisse.
